# EUROPÄISCHE PATENTSCHRIFT

(11) **EP 2 076 486 B1**
(45) Veröffentlichungstag und Bekanntmachung des Hinweises auf die Patenterteilung: **12.12.2012**
(21) Anmeldenummer: 07818756.4
(22) Anmeldetag: 05.10.2007
(51) Int. Cl.: C07C 231/02, C07C 233/05, C07C 233/38

(54) **VERFAHREN ZUR HERSTELLUNG BASISCHER FETTSÄUREAMIDE**
METHOD FOR PRODUCING ALKALINE FATTY ACID AMIDES
PROCEDE DE PRODUCTION D'AMIDES D'ACIDES GRAS BASIQUES

(30) Priorität: 09.10.2006 DE 102006047619
(43) Veröffentlichungstag der Anmeldung: 08.07.2009
(73) Patentinhaber: Clariant Finance (BVI) Limited, Road Town, Tortola (VG)
(72) Erfinder: KRULL, Matthias, 55296 Harxheim (DE); MORSCHHÄUSER, Roman, 55122 Mainz (DE); KAYSER, Christoph, 55127 Mainz (DE); RITTER, Helmut, 42111 Wuppertal (DE); SCHMITZ, Sarah, 45130 Essen (DE)
(74) Vertreter: Mikulecky, Klaus
(86) Internationale Anmeldenummer: PCT/EP2007/008680
(87) Internationale Veröffentlichungsnummer: WO 2008/043495

(56) Entgegenhaltungen:
- WO-A-2005/118526
- DE-B- 1 139 738
- US-A1- 2005 283 011

## Beschreibung

Fettsäurederivate, die funktionelle Gruppen mit basischem Charakter tragen, sind als Vorstufen zur Herstellung oberflächenaktiver Substanzen begehrt. Durch Umsetzung mit Alkylierungsmitteln sind sie in kationische Tenside überführbar. Durch Umsetzung mit saure Gruppen tragenden Alkylierungsmitteln sind aus ihnen so genannte Betaine zugänglich; Oxidationsreaktionen mit Peroxiden führen zur Gruppe der Aminoxide, einer ebenfalls als amphoter anzusehender Produktgruppe. Aminoxide und Betaine finden in großem Ausmaß Verwendung als Rohstoffe für die Herstellung von Waschmitteln, Reinigungskonzentraten, Detergentien, Kosmetika und Pharmazeutika, als Emulgatoren sowie in der Erdölindustrie als Korrosions- oder Gashydratinhibitoren.

Von besonderem Interesse sind dabei insbesondere solche Fettsäurederivate, die einen über eine Amidgruppe verknüpften Alkylrest tragen, der seinerseits mit mindestens einer basischen Charakter verleihenden tertiären Aminogruppe substituiert ist. Derartige Amide weisen eine gegenüber entsprechenden Estern stark erhöhte Hydrolysestabilität auf. Basische Fettsäureamide werden üblicherweise nach weiterer Umsetzung beispielsweise zu quaternären Ammoniumverbindungen, N-Oxiden oder auch Betainen in oben genannten Anwendungen eingesetzt.

US 2005/0283011 offenbart eine Methode zun Herstellung von Fettsäune a mildew.

Um den wachsenden Bedarf für bestehende und neue Anwendungen zu decken, wurden verschiedene Methoden für die Herstellung tertiäre Aminogruppen tragender Fettsäureamide entwickelt. Bei der Herstellung derartiger basischer Amide ist man dabei bisher auf kostenintensive und/oder langwierige Herstellverfahren angewiesen, um eine kommerziell interessante Ausbeute zu erzielen. Die bekannten Herstellverfahren erfordern aktivierte Carbonsäurederivate wie beispielsweise Säureanhydride, Säurehalogenide wie Säurechloride oder Ester bzw. eine in-situ-Aktivierung durch den Einsatz von Kupplungsreagentien wie beispielsweise N,N'-Dicyclohexylcarbodiimid. Bei diesen Herstellverfahren entstehen zum Teil große Mengen unerwünschter Nebenprodukte wie Alkohole, Säuren und Salze, die vom Produkt abgetrennt und entsorgt werden müssen. Aber auch die in den Produkten verbleibenden Reste dieser Hilfs- und Nebenprodukte können zum Teil sehr unerwünschte Effekte bewirken. So führen beispielsweise Halogenidionen wie auch Säuren zu Korrosion; Kupplungsreagentien sowie die von ihnen gebildeten Nebenprodukte sind zum Teil giftig, sensibilisierend oder auch carcinogen.

Die direkte thermische Kondensation von Carbonsäure und Diamin führt zu keinen befriedigenden Resultaten, da verschiedene Nebenreaktionen die Ausbeute mindern. Dazu zählen beispielsweise eine Decarboxylierung der Carbonsäure, eine Oxidation der Aminogruppe während des zur Erzielung hoher Umsätze erforderlichen langen Erhitzens und insbesondere der thermisch induzierte Abbau der tertiären Aminogruppe. Da solche Nebenreaktionen unter anderem zur Bildung reaktionsfreudiger C=C-Doppelbindungen führen, können auf diesem Weg sowohl aus dem eingesetzten Amin wie auch aus den einmal gebildeten Amiden Verbindungen mit polymerisationsfähigen Zentren entstehen, die zu unerwünschter Polymerbildung und anderen Nebenreaktionen führen. So können beispielsweise aus N-[3-(N,N-Dimethylamino)propyl]fettsäureamiden in Gegenwart von Säuren durch Hofmann-Abbau N-(Allyl)fettsäureamide entstehen. Nebenreaktionen führen zudem zu gefärbten Nebenprodukten und es ist in der Folge nicht möglich, insbesondere für kosmetische Anwendungen gewünschte farblose Produkte mit Jodfarbzahlen von beispielsweise weniger als 6 herzustellen. Letzteres erfordert entweder den Einsatz von farbverbessernden Additiven während der thermischen Amidierungsreaktion und/oder zusätzliche Verfahrensschritte wie beispielsweise das Bleichen, das aber seinerseits den Zusatz weiterer Hilfsstoffe erfordert und oftmals zu einer ebenso unerwünschten Beeinträchtigung des Geruchs der Amide führt.

Goretzki et.al., Macromol. Rapid Commun. 2004, 25, 513-516 offenbart die durch Mikrowellen unterstützte Synthese verschiedener (Meth)acrylamide direkt aus (Meth)acrylsäure und Amin. Es werden verschiedene aliphatische und aromatische Amine eingesetzt.

Gelens et al., Tetrahedron Letters 2005, 46(21), 3751-3754 offenbart eine Vielzahl an Amiden, die unter Zuhilfenahme von Mikrowellenstrahlung synthetisiert wurden. Keines enthält jedoch eine zusätzliche tertiäre Aminogruppe.

Es wurde folglich ein Verfahren zur Herstellung von basischen Fettsäureamiden gesucht, bei dem Fettsäuren und tertiäre Aminogruppen tragende Amine direkt und in hohen, das heißt bis zu quantitativen Ausbeuten zu tertiäre Aminogruppen tragenden Fettsäureamiden umgesetzt werden können. Weiterhin sollen dabei keine bzw. nur untergeordnete Mengen an Nebenprodukten wie insbesondere ethylenisch ungesättigte Verbindungen und deren Folgeprodukte anfallen. Weiterhin sollen dabei basische Fettsäureamide mit möglichst geringer Eigenfärbung entstehen.

Es wurde gefunden, dass sich tertiäre Aminogruppen tragende Fettsäureamide durch direkte Umsetzung von mindestens eine primäre oder sekundäre Aminogruppe und zusätzlich mindestens eine tertiäre Aminogruppe tragenden Polyaminen mit Fettsäuren durch Bestrahlung mit Mikrowellen in hohen Ausbeuten herstellen lassen. Überraschenderweise treten dabei trotz der Anwesenheit von Säuren keine nennenswerten Nebenreaktionen und insbesondere keine Hofmann-Eliminierung der tertiären Aminogruppe auf. Weiterhin zeigen die so hergestellten Fettsäureamide eine im Vergleich zu nach konventionellen Herstellverfahren ohne zusätzliche Verfahrensschritte nicht zugängliche niedrige Eigenfärbung.

Gegenstand der Erfindung ist ein Verfahren zur Herstellung von basischen Fettsäureamiden, indem mindestens ein Amin, das mindestens eine primäre oder sekundäre Aminogruppe und mindestens eine tertiäre Aminogruppe enthält, mit mindestens einer Fettsäure zu einem Ammoniumsalz umgesetzt wird, und dieses Ammoniumsalz nachfolgend unter Mikrowellenbestrahlung weiter zum basischen Amid umgesetzt wird.

Ein weiterer Gegenstand der Erfindung sind basische Fettsäureamide, herstellbar, indem mindestens ein Amin, das mindestens eine primäre oder sekundäre Aminogruppe und mindestens eine tertiäre Aminogruppe enthält, mit mindestens einer Fettsäure zu einem Ammoniumsalz umgesetzt wird, und dieses Ammoniumsalz nachfolgend unter Mikrowellenbestrahlung weiter zum basischen Amid umgesetzt wird.

Ein weiterer Gegenstand der Erfindung sind basische Fettsäureamide, die Jodfarbzahlen von weniger als 5 aufweisen, herstellbar, indem mindestens ein Amin, das mindestens eine primäre oder sekundäre Aminogruppe und mindestens eine tertiäre Aminogruppe enthält, mit mindestens einer Fettsäure zu einem Ammoniumsalz umgesetzt wird, und dieses Ammoniumsalz nachfolgend unter Mikrowellenbestrahlung weiter zum basischen Amid umgesetzt wird.

Ein weiterer Gegenstand der Erfindung sind basische Fettsäureamide, die von Halogenidionen und aus Kupplungsreagentien stammenden Nebenprodukten frei sind, herstellbar, indem mindestens ein Amin, das mindestens eine primäre oder sekundäre Aminogruppe und mindestens eine tertiäre Aminogruppe enthält, mit mindestens einer Fettsäure zu einem Ammoniumsalz umgesetzt wird, und dieses Ammoniumsalz nachfolgend unter Mikrowellenbestrahlung weiter zum basischen Amid umgesetzt wird.

Unter basischen Amiden werden Amide verstanden, deren Amidstickstoffatom mindestens einen mit mindestens einer tertiären Aminogruppe substituierten Kohlenwasserstoffrest trägt. Tertiäre Aminogruppen im Sinne der vorliegenden Erfindung sind Struktureinheiten, in denen ein Stickstoffatom kein acides Proton trägt. So kann der Stickstoff der tertiären Aminogruppe drei Kohlenwasserstoffreste tragen oder auch Bestandteil eines heteroaromatischen Systems sein. Von Halogenidionen freie Fettsäureamide enthalten keine über die ubiquitären Mengen an Halogenidionen hinausgehenden Mengen dieser Ionen.

Unter Fettsäuren werden vorzugsweise Carbonsäuren verstanden, die einen Kohlenwasserstoffrest mit 1 bis 50 C-Atomen tragen. Bevorzugte Fettsäuren haben 6 bis 50, insbesondere 8 bis 30 und speziell 10 bis 24 C-Atome wie beispielsweise 12 bis 18 C-Atome. Sie können natürlichen oder synthetischen Ursprungs sein. Sie können Substituenten wie beispielsweise Halogenatome, halogenierte Alkylreste, Cyano-, Hydroxyalkyl-, Hydroxyl-, Methoxy-, Nitril-, Nitro-, und/oder Sulfonsäuregruppen tragen. Besonders bevorzugt sind aliphatische Kohlenwasserstoffreste. Diese aliphatischen Kohlenwasserstoffreste können linear, verzweigt oder zyklisch sowie gesättigt oder ungesättigt sein. Sind sie ungesättigt, so können sie eine oder mehrere wie beispielsweise zwei, drei oder mehr Doppelbindungen enthalten. Bevorzugt befindet sich keine Doppelbindung in α,β-Position zur Carboxylgruppe. Geeignete Fettsäuren sind beispielsweise Octan-, Decan-, Dodecan-, Tridecan-, Tetradecan-, 12-Methyltridecan-, Pentadecan-, 13-Methyltetradecan-, 12-Methyltetradecan-, Hexadecan-, 14-Methylpentadecan-, Heptadecan-, 15-Methylhexadecan-, 14-Methylhexadecan- , Octadecan-, Iso-Octadecan-, Icosan-, Docosan- und Tetracosansäure sowie Myristolein-, Palmitolein-, Hexadecadien-, Delta-9-cis-Heptadecen-, Öl-, Petroselin-, Vaccen-, Linol-, Linolen-, Gadolein-, Gondo-, Icosadien-, Arachidon-, Cetolein-, Eruca-, Docosadien- und Tetracosensäure sowie Ricinolsäure. Weiterhin geeignet sind aus natürlichen Fetten und Ölen wie beispielsweise Baumwollsamen-, Cocos-, Erdnuss-, Färberdistel-, Mais-, Palmkern-, Raps-, Ricinus-, Oliven-, Senfsamen, Soja-, Sonnenblumenöl sowie Talg-, Knochen- und Fischöl gewonnene Fettsäuremischungen. Als Fettsäuren bzw. Fettsäuremischungen für das erfindungsgemäße Verfahren ebenfalls geeignet sind Tallölfettsäure sowie Harz- und Naphthensäuren.

Erfindungsgemäß geeignete Amine besitzen zwei oder mehr Aminogruppen. Von diesen Aminogruppen ist mindestens eine tertiär, das heißt dass sie drei Alkylreste trägt oder Bestandteil eines heteroaromatischen Systems ist. Von diesen Aminogruppen ist weiterhin mindestens eine primäre oder sekundäre Aminogruppe, das heißt mindestens eine Aminogruppe trägt ein oder zwei Wasserstoffatome. Bevorzugt ist diese Aminogruppe eine primäre Aminogruppe, das heißt sie trägt zwei Wasserstoffatome. In einer weiteren bevorzugten Ausführungsform enthält das erfindungsgemäß geeignete Amin drei oder mehr Aminogruppen, von denen mindestens eine primär, mindestens eine sekundär und mindestens eine tertiär ist. Bevorzugte Amine entsprechen der Formel

HNR¹-(A)ₙ-Z

worin
- R¹: für Wasserstoff, C₁-C₁₂-Alkyl, C₅-C₁₂-Cycloalkyl, C₆-C₁₂-Aryl, C₇-C₃₀-Aralkyl oder eine heteroaromatische Gruppe mit 5 bis 12 Ringgliedern,
- A: für einen zweiwertigen Kohlenwasserstoffrest mit 2 bis 50 C-Atomen,
- n: für 0 oder 1,
- Z: für eine Gruppe der Formel -NR²R³ oder für einen Stickstoff enthaltenden zyklischen Kohlenwasserstoffrest mit mindestens 5 Ringgliedern und
- R² und R³: unabhängig voneinander für C₁- bis C₂₀-Kohlenwasserstoffreste stehen.

Bevorzugt steht R¹ für Wasserstoff oder Methyl, insbesondere für Wasserstoff.

Bevorzugt steht A für einen Alkylenrest mit 2 bis 50 C-Atomen, einen Cycloalkylenrest mit 5 bis 12 Ringgliedern, einen Arylenrest mit 6 bis 12 Ringgliedern oder einen Heteroarylenrest mit 5 bis 12 Ringgliedern. Besonders bevorzugt steht A für einen Alkylenrest mit 2 bis 12 C-Atomen. Bevorzugt steht n für 1. Besonders bevorzugt stehen A für einen linearen oder verzweigten Alkylenrest mit 1 bis 12 C-Atomen und n für 1.

Besonders bevorzugt steht A, wenn Z für eine Gruppe der Formel -NR²R³ steht, für einen linearen oder verzweigten Alkylenrest mit 2, 3 oder 4 C-Atomen, insbesondere für einen Ethylenrest oder einen linearen Propylenrest. Steht Z dagegen für einen Stickstoff enthaltenden zyklischen Kohlenwasserstoffrest, so sind Verbindungen besonders bevorzugt, in denen A für einen linearen Alkylenrest mit 1, 2 oder 3 C-Atomen, insbesondere für einen Methylen-, Ethylen- oder einen linearen Propylenrest steht.

Für das Strukturelement A bevorzugte zyklische Reste können mono- oder polyzyklisch sein und beispielsweise zwei oder drei Ringsysteme enthalten. Bevorzugte Ringsysteme besitzen 5, 6 oder 7 Ringglieder. Bevorzugt enthalten sie insgesamt etwa 5 bis 20 C-Atome, insbesondere 6 bis 10 C-Atome. Bevorzugte Ringsysteme sind aromatisch und enthalten nur C-Atome. In einer speziellen Ausführungsform werden die Strukturelemente A aus Arylenresten gebildet. Das Strukturelement A kann Substituenten wie beispielsweise Alkylreste, Halogenatome, halogenierte Alkylreste, Nitro-, Cyano-, Nitril-, Hydroxyl- und/oder Hydroxyalkylgruppen tragen. Ist A ein monozyklischer aromatischer Kohlenwasserstoff, so befinden sich die Aminogruppen bzw. Aminogruppen tragenden Substituenten bevorzugt in ortho- oder para-Stellung zueinander.

Bevorzugt steht Z für eine Gruppe der Formel -NR²R³. Darin stehen R² und R³ unabhängig voneinander bevorzugt für aliphatische, aromatische und/oder araliphatische Kohlenwasserstoffreste mit 1 bis 20 Kohlenstoffatomen. Als R² und R³ besonders bevorzugt sind Alkylreste. Sind R² und/oder R³ Alkylreste, so tragen sie bevorzugt 1 bis 14 C-Atome wie beispielsweise 1 bis 6 C-Atome. Diese Alkylreste können linear, verzweigt und/oder zyklisch sein. Besonders bevorzugt stehen R² und R³ für Alkylreste mit 1 bis 4 C-Atomen wie beispielsweise Methyl, Ethyl, n-Propyl, iso-Propyl, n-Butyl und iso-Butyl.

Die Reste R² und R³ können durch Heteroatome wie beispielsweise O und/oder S substituiert sein, und/oder solche Heteroatome enthaltende Substituenten tragen. Bevorzugt enthalten sie jedoch nicht mehr als 1 Heteroatom pro 2 C-Atome. So stehen In einer weiteren bevorzugten Ausführungsform R² und/oder R³ unabhängig voneinander für Polyoxyalkylenreste der Formel

-(B-O)ₘ-R⁴,

worin
- B: für einen linearen oder verzweigten C₂-C₄-Alkylenrest, insbesondere für eine Gruppe der Formel -CH₂-CH₂- und/oder -CH(CH₃)-CH₂-,
- m: für eine Zahl von 1 bis 100, bevorzugt 2 bis 20 und
- R⁴: für Wasserstoff, einen Alkylrest mit 1 bis 20 C-Atomen, einen Cycloalkylrest mit 5 bis 12 Ringatomen, einen Arylrest mit 6 bis 12 Ringatomen, einen Aralkylrest mit 7 bis 30 C-Atomen, einen Heteroarylrest mit 5 bis 12 Ringatomen oder einen Hetero-Aralkylrest mit 6 bis 12 C-Atomen steht.

Als R² und/oder R³ besonders geeignete aromatische Reste umfassen Ringsysteme mit mindestens 5 Ringgliedern. Sie können Heteroatome wie S, O und N enthalten. Als R² und/oder R³ besonders geeignete araliphatische Reste umfassen Ringsysteme mit mindestens 5 Ringgliedern, die über einen C₁-C₆-Alkylrest an den Stickstoff gebunden sind. Sie können Heteroatome wie S, O und N enthalten. Die aromatischen wie auch die araliphatischen Reste können weitere Substituenten wie beispielsweise Alkylreste, Halogenatome, halogenierte Alkylreste, Nitro-, Cyano-, Nitril-, Hydroxyl- und/oder Hydroxyalkylgruppen tragen.

In einer weiteren bevorzugten Ausführungsform steht Z für einen Stickstoff enthaltenden, zyklischen Kohlenwasserstoffrest, dessen Stickstoffatom nicht zur Bildung von Amiden befähigt ist. Das zyklische System kann mono-, di- oder auch polyzyklisch sein. Bevorzugt enthält es einen oder mehrere fünf- und/oder sechsgliedrige Ringe. Dieser zyklische Kohlenwasserstoff kann ein oder mehrere wie beispielsweise zwei oder drei Stickstoffatome enthalten, die keine aciden Protonen tragen, besonders bevorzugt enthält er ein N-Atom. Besonders geeignet sind dabei stickstoffhaltige Aromaten, deren Stickstoff an der Ausbildung eines aromatischen π-Elektronensextetts beteiligt ist wie beispielsweise Pyridin. Gleichfalls geeignet sind stickstoffhaltige Heteroaliphaten, deren Stickstoffatome keine Protonen tragen und zum Beispiel sämtlich mit Alkylresten abgesättigt sind. Die Verknüpfung von Z mit A bzw. der Gruppe der Formel NHR¹ (falls n = 0) erfolgt hier bevorzugt über ein Stickstoffatom des Heterozyklus wie beispielsweise bei 1-(3-Aminopropyl)pyrrolidin. Der durch Z repräsentierte zyklische Kohlenwasserstoff kann weitere Substituenten wie beispielsweise C₁-C₂₀-Alkylreste, Halogenatome, halogenierte Alkylreste, Nitro-, Cyano-, Nitril-, Hydroxyl - und/oder Hydroxyalkylgruppen tragen.

Beispiele für erfindungsgemäß geeignete Amine sind N,N-Dimethylethylendiamin, N,N-Dimethyl-1,3-propandiamin, N,N-Diethyl-1,3-propandiamin, N,N-Dimethyl-2-methyl-1,3-propandiamin, N,N-(2'-hydroxyethyl)-1,3-propandiamin, 1-(3-Aminopropyl)pyrrolidin, 1-(3-Aminopropyl)-4-methylpiperazin, 3-(4-Morpholino)-1-propylamin, 2-Aminothiazol, die verschiedenen Isomere des N,N-Dimethylamino-anilins, des Aminopyridins, des Aminomethylpyridins, des Aminomethylpiperidins und des Aminochinolins, sowie 2-Aminopyrimidin, 3-Aminopyrazol, Aminopyrazin und 3-Amino-1,2,4-triazol.

Das Verfahren ist insbesondere geeignet zur Herstellung von
N-(N',N'-Dimethylamino)propyl-docecansäureamid,
N-(N',N'-Dimethylamino)propyl-cocosfettsäureamid,
N-(N',N'-Dimethylamino)propyl-talgfettsäureamid, N-(N',N'-Dimethylamino)ethyl-cocosfettsäureamid, und N-(N',N'-Dimethylamino)propyl-palmfettsäureamid.

Im erfindungsgemäßen Verfahren können Fettsäure und Amin in beliebigen Verhältnissen miteinander zur Reaktion gebracht werden. Bevorzugt erfolgt die Umsetzung mit molaren Verhältnissen zwischen Fettsäure und Amin von 10:1 bis 1:10, bevorzugt von 2:1 bis 1:2, speziell von 1:1,2 bis 1,2:1 und insbesondere equimolar.

In vielen Fällen hat es sich als vorteilhaft erwiesen, mit einem geringen Überschuss an Amin, das heißt molaren Verhältnissen von Amin zu Fettsäure von mindestens 1,01 : 1,00 und insbesondere zwischen 1,02 : 1,00 und 1,3 : 1,0 wie beispielsweise zwischen 1,05 : 1,0 und 1,1 : 1 zu arbeiten. Dabei wird die Fettsäure praktisch quantitativ zum basischen Amid umgesetzt. Besonders vorteilhaft ist dieses Verfahren, wenn das eingesetzte, mindestens eine primäre und/oder sekundäre und mindestens eine tertiäre Aminogruppe tragende Amin leicht flüchtig ist. Leicht flüchtig heißt hier, dass das Amin einen Siedepunkt bei Normaldruck von vorzugsweise unterhalb 200°C wie beispielsweise unterhalb 150°C besitzt und sich somit destillativ vom Amid abtrennen lässt.

Die erfindungsgemäße Herstellung der Amide erfolgt durch Umsetzung der Fettsäure und des Amins zum Ammoniumsalz und nachfolgender Bestrahlung des Salzes mit Mikrowellen. Das Ammoniumsalz wird dabei bevorzugt in-situ erzeugt und nicht isoliert. Bevorzugt wird der durch die Mikrowellenbestrahlung bedingte Temperaturanstieg durch Regelung der Mikrowellenintensität und/oder Kühlung des Reaktionsgefäßes auf maximal 300°C begrenzt. Besonders bewährt hat sich die Durchführung der Umsetzung bei Temperaturen zwischen 100 und maximal 250°C und speziell zwischen 120 und maximal 200°C wie beispielsweise bei Temperaturen zwischen 125 und 190°C.

Die Dauer der Mikrowellenbestrahlung hängt von verschiedenen Faktoren wie dem Reaktionsvolumen, der Geometrie des Reaktionsraumes und dem gewünschten Umsetzungsgrad ab. Üblicherweise wird die Mikrowellenbestrahlung über einen Zeitraum von weniger als 30 Minuten, bevorzugt zwischen 0,01 Sekunden und 15 Minuten, besonders bevorzugt zwischen 0,1 Sekunden und 10 Minuten und insbesondere zwischen einer Sekunde und 5 Minuten wie beispielsweise zwischen 5 Sekunden und 2 Minuten vorgenommen. Die Intensität (Leistung) der Mikrowellenstrahlung wird dabei so eingestellt, dass das Reaktionsgut in möglichst kurzer Zeit die angestrebte Reaktionstemperatur erreicht. Zum anschließenden Aufrechterhalten der Temperatur kann das Reaktionsgut mit reduzierter und/oder gepulster Leistung weiter bestrahlt werden. Zur Einhaltung der Maximaltemperatur bei gleichzeitig größtmöglicher Mikrowelleneinstrahlung hat es sich bewährt, das Reaktionsgut beispielsweise mittels Kühlmantel, im Reaktionsraum befindliche Kühlrohre, durch intermittierende Kühlung zwischen verschiedenen Bestrahlungszonen und/oder durch Siedekühlung über externe Wärmetauscher zu kühlen. In einer bevorzugten Ausführungsform wird das Umsetzungsprodukt direkt nach Beendigung der Mikrowellenbestrahlung möglichst schnell auf Temperaturen unterhalb 120°C, bevorzugt unterhalb 100°C und speziell unterhalb 60°C abgekühlt.

Bevorzugt wird die Umsetzung bei Drücken zwischen 0,01 und 200 bar und speziell zwischen 1 bar (Atmosphärendruck) und 50 bar durchgeführt. Besonders bewährt hat sich das Arbeiten in geschlossenen Gefäßen, in denen oberhalb des Siedepunkts der Edukte bzw. Produkte, des gegebenenfalls anwesenden Lösemittels und/oder oberhalb des während der Reaktion gebildeten Reaktionswassers gearbeitet wird. Üblicherweise reicht der sich auf Grund der Erwärmung des Reaktionsansatzes einstellende Druck zur erfolgreichen Durchführung des erfindungsgemäßen Verfahrens aus. Es kann aber auch unter erhöhtem Druck und/oder unter Anlegen eines Druckprofils gearbeitet werden. In einer weiteren bevorzugten Variante des erfindungsgemäßen Verfahrens wird unter Atmosphärendruck, wie er sich zum Beispiel im offenen Gefäß einstellt, gearbeitet.

Zur Vermeidung von Nebenreaktionen und zur Herstellung von möglichst reinen Produkten hat es sich bewährt, das erfindungsgemäße Verfahren in Gegenwart eines inerten Schutzgases wie beispielsweise Stickstoff, Argon oder Helium durchzuführen.

In einer bevorzugten Ausführungsform wird zur Beschleunigung bzw. zur Vervollständigung der Reaktion in Gegenwart von dehydratisierenden Katalysatoren gearbeitet. Vorzugsweise arbeitet man dabei in Gegenwart eines sauren anorganischen, metallorganischen oder organischen Katalysators oder Gemischen aus mehreren dieser Katalysatoren.

Als saure anorganische Katalysatoren im Sinne der vorliegenden Erfindung sind beispielsweise Schwefelsäure, Phosphorsäure, Phosphonsäure, hypophosphorige Säure, Aluminiumsulfathydrat, Alaun, saures Kieselgel und saures Aluminiumhydroxid zu nennen. Weiterhin sind beispielsweise Aluminiumverbindungen der allgemeinen Formel Al(OR⁵)₃ und Titanate der allgemeinen Formel Ti(OR⁵)₄ als saure anorganische Katalysatoren einsetzbar, wobei die Reste R⁵ jeweils gleich oder verschieden sein können und unabhängig voneinander gewählt sind aus C₁-C₁₀-Alkylresten, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, sec.-Hexyl, n-Heptyl, n-Octyl, 2-Ethylhexy, n-Nonyl oder n-Decyl, C₃-C₁₂-Cycloalkylresten, beispielsweise Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl. Bevorzugt sind die Reste R⁵ in Al(OR⁵)₃ bzw. Ti(OR⁵)₄ jeweils gleich und gewählt aus Isopropyl, Butyl und 2-Ethylhexyl.

Bevorzugte saure metallorganische Katalysatoren sind beispielsweise gewählt aus Dialkylzinnoxiden (R⁵)₂SnO, wobei R⁵ wie oben stehend definiert ist. Ein besonders bevorzugter Vertreter für saure metallorganische Katalysatoren ist Di-n-butylzinnoxid, das als so genanntes Oxo-Zinn oder als Fascat^{®}-Marken kommerziell erhältlich ist.

Bevorzugte saure organische Katalysatoren sind saure organische Verbindungen mit beispielsweise Phosphatgruppen, Sulfonsäuregruppen, Sulfatgruppen oder Phosphonsäuregruppen. Besonders bevorzugte Sulfonsäuren enthalten mindestens eine Sulfonsäuregruppe und mindestens einen gesättigten oder ungesättigten, linearen, verzweigten und/oder zyklischen Kohlenwasserstoffrest mit 1 bis 40 C-Atomen und bevorzugt mit 3 bis 24 C-Atomen. Insbesondere bevorzugt sind aromatische Sulfonsäuren, speziell alkylaromatische Mono-Sulfonsäuren mit einem oder mehreren C₁-C₂₈-Alkylresten und insbesondere solche mit C₃-C₂₂-Alkylresten. Geeignete Beispiele sind Methansulfonsäure, Butansulfonsäure, Benzolsulfonsäure, p-Toluolsulfonsäure, Xylolsulfonsäure, 2-Mesitylensulfonsäure, 4-Ethylbenzolsulfonsäure, Isopropylbenzolsulfonsäure, 4-Butylbenzolsulfonsäure, 4-Octylbenzolsuffonsäure; Dodecylbenzolsulfonsäure, Didodecylbenzolsulfonsäure, Naphthalinsulfonsäure. Auch saure Ionenaustauscher können als saure organische Katalysatoren eingesetzt werden, beispielsweise Sulfonsäuregruppenhaltige Poly(styrol)-Harze, die mit etwa 2 mol-% Divinylbenzol vernetzt sind.

Besonders bevorzugt für die Durchführung des erfindungsgemäßen Verfahrens sind Borsäure, Phosphorsäure, Polyphosphorsäure und Polystyrolsulfonsäuren. Insbesondere bevorzugt sind Titanate der allgemeinen Formel Ti(OR⁵)₄ und speziell Titantetrabutylat und Titantetraisopropylat.

Wünscht man saure anorganische, metallorganische oder organische Katalysatoren einzusetzen, so setzt man erfindungsgemäß 0,01 bis 10 Gew.-%, bevorzugt 0,02 bis 2 Gew.-% Katalysator ein. In einer besonders bevorzugten Ausführungsform wird ohne Katalysator gearbeitet.

In einer weiteren bevorzugten Ausführungsform wird die Mikrowellenbestrahlung in Gegenwart von sauren, festen Katalysatoren durchgeführt. Dabei wird der feste Katalysator in dem gegebenenfalls mit Lösemittel versetzten Ammoniumsalz suspendiert oder bei kontinuierlichen Verfahren Vorteilhafterweise das gegebenenfalls mit Lösemittel versetzte Ammoniumsalz über einen Festbettkatalysator geleitet und Mikrowellenstrahlung ausgesetzt. Geeignete feste Katalysatoren sind beispielsweise Zeolithe, Kieselgel, Montmorillonit und (teil)vernetzte Polystyrolsulfonsäure, die gegebenenfalls mit katalytisch aktiven Metallsalzen imprägniert sein können. Geeignete saure Ionentauscher auf Basis von Polystyrolsulfonsäuren, die als Festphasenkatalysatoren eingesetzt werden können, sind beispielsweise von der Firma Rohm & Haas unter der Markenbezeichnung Amberlyst^{®} erhältlich.

Es hat sich bewährt, in Gegenwart von Lösemitteln zu arbeiten um beispielsweise die Viskosität des Reaktionsmediums abzusenken, das Reaktionsgemisch, sofern es heterogen ist, zu fluidisieren und/oder die Wärmeabfuhr beispielsweise mittels Siedekühlung zu verbessern. Dafür können prinzipiell alle Lösemittel eingesetzt werden, die unter den angewendeten Reaktionsbedingungen inert sind und nicht mit den Edukten bzw. den gebildeten Produkten reagieren. Ein wichtiger Faktor bei der Auswahl geeigneter Lösemittel ist deren Polarität, die einerseits die Löseeigenschaften und andererseits das Ausmaß der Wechselwirkung mit Mikrowellenstrahlung bestimmt. Ein besonders wichtiger Faktor bei der Auswahl geeigneter Lösemittel ist deren dielektrischer Verlust ε". Der dielektrische Verlust ε" beschreibt den Anteil an Mikrowellenstrahlung, der bei der Wechselwirkung einer Substanz mit Mikrowellenstrahlung in Wärme überführt wird. Letzt genannter Wert hat sich als besonders wichtiges Kriterium für die Eignung eines Lösemittels für die Durchführung des erfindungsgemäßen Verfahrens erwiesen. Besonders bewährt hat sich das Arbeiten in Lösemitteln, die eine möglichst geringe Mikrowellenabsorption zeigen und somit nur einen kleinen Beitrag zur Erwärmung des Reaktionssystems liefern. Für das erfindungsgemäße Verfahren bevorzugte Lösemittel besitzen einen bei Raumtemperatur und 2450 MHz gemessenen dielektrischen Verlust ε" von weniger als 10 und vorzugsweise weniger als 1 wie beispielsweise weniger als 0,5. Eine Übersicht über den dielektrischen Verlust verschiedener Lösemittel findet sich zum Beispiel in "Microwave Synthesis" von B. L. Hayes, CEM Publishing 2002. Für das erfindungsgemäße Verfahren geeignet sind insbesondere Lösemittel mit ε"-Werten unterhalb 10 wie N-Methylpyrrolidon, N,N-Dimethylformamid oder Aceton, und insbesondere Lösemittel mit ε"-Werten unterhalb 1. Beispiele für besonders bevorzugte Lösemittel mit ε"-werten unterhalb 1 sind aromatische und/oder aliphatische Kohlenwasserstoffe wie beispielsweise Toluol, Xylol, Ethylbenzol, Tetralin, Hexan, Cyclohexan, Decan, Pentadecan, Dekalin sowie kommerzielle Kohlenwasserstoffgemische wie Benzinfraktionen, Kerosin, Solvent Naphtha, ^{®}Shellsol AB, ^{®}Solvesso 150, ^{®}Solvesso 200, ^{®}Exxsol, ^{®}Isopar und ^{®}Shellsol-Typen. Lösemittelgemische, die ε"-Werte bevorzugt unterhalb 10 und speziell unterhalb 1 aufweisen, sind für die Durchführung des erfindungsgemäßen Verfahrens gleichermaßen bevorzugt. Prinzipiell ist das erfindungsgemäße Verfahren auch in Lösemitteln mit ε"-Werten von 10 und höher möglich, doch erfordert dies besondere Maßnahmen zur Einhaltung der Maximaltemperatur und führt oftmals zu verminderten Ausbeuten. Sofern in Gegenwart von Lösemitteln gearbeitet wird, liegt deren Anteil an der Reaktionsmischung bevorzugt zwischen 2 und 95 Gew.-%, speziell zwischen 5 und 90 Gew.% und insbesondere zwischen 10 und 75 Gew.-% wie beispielsweise zwischen 30 und 60 Gew.-%. Besonders bevorzugt wird die Reaktion lösemittelfrei durchgeführt.

Die Mikrowellenbestrahlung wird üblicherweise in Geräten durchgeführt, die einen Reaktionsraum aus einem für Mikrowellen weitestgehend transparenten Material besitzen, in den in einem Mikrowellengenerator erzeugte Mikrowellenstrahlung über geeignete Antennensysteme eingekoppelt wird. Mikrowellengeneratoren, wie beispielsweise das Magnetron und das Klystron sind dem Fachmann bekannt.

Als Mikrowellen werden elektromagnetische Strahlen mit einer Wellenlänge zwischen etwa 1 cm und 1 m und Frequenzen zwischen etwa 300 MHz und 30 GHz bezeichnet. Dieser Frequenzbereich ist prinzipiell für das erfindungsgemäße Verfahren geeignet. Bevorzugt wird für das erfindungsgemäße Verfahren Mikrowellenstrahlung mit den für industrielle, wissenschaftliche und medizinische Anwendungen freigegebenen Frequenzen von 915 MHz, 2,45 GHz, 5,8 GHz oder 27,12 GHz verwendet. Es kann sowohl im Mono- bzw. Quasi-Monomode wie auch im Multimode gearbeitet werden. Beim Monomode, der hohe Anforderungen an Geometrie und Größe von Apparatur und Reaktionsraum stellt, wird dabei durch eine stehende Welle insbesondere an deren Maximum eine sehr hohe Energiedichte erzeugt. Beim Multimode dagegen wird der gesamte Reaktionsraum weitgehend homogen bestrahlt, was zum Beispiel größere Reaktionsvolumina ermöglicht.

Die für die Durchführung des erfindungsgemäßen Verfahrens in das Reaktionsgefäß einzustrahlende Mikrowellenleistung ist insbesondere abhängig von der Geometrie des Reaktionsraums und damit des Reaktionsvolumens sowie der Dauer der erforderlichen Bestrahlung. Sie liegt üblicherweise zwischen 100 W und mehreren 100 kW und insbesondere zwischen 200 W und 100 kW wie beispielsweise zwischen 500 W und 70 kW. Sie kann an einer oder mehreren Stellen des Reaktors appliziert werden. Sie kann über einen oder mehrere Mikrowellengeneratoren erzeugt werden.

Die Reaktion kann diskontinuierlich im Batch-Verfahren oder, bevorzugt, kontinuierlich zum Beispiel in einem Strömungsrohr durchgeführt werden. Sie kann weiterhin in semi-Batch Prozessen wie beispielsweise kontinuierlich betriebenen Rührreaktoren oder Kaskadenreaktoren durchgeführt werden. In einer bevorzugten Ausführungsform wird die Reaktion in einem geschlossenen Gefäß durchgeführt, wobei das sich bildende Kondensat sowie gegebenenfalls Edukte und, sofern anwesend, Lösemittel zu einem Druckaufbau führen. Nach Beendigung der Reaktion kann der Überdruck durch Entspannung zur Verflüchtigung und Abtrennung von Reaktionswasser und gegebenenfalls Lösemittel sowie überschüssigen Edukten und/oder Abkühlung des Reaktionsprodukts verwendet werden. In einer weiteren Ausführungsform wird das gebildete Reaktionswasser nach dem Abkühlen und/oder Entspannen durch übliche Verfahren wie beispielsweise Phasentrennung, Destillation und/oder Absorption abgetrennt. Das erfindungsgemäße Verfahren kann ebenso erfolgreich in einem offenen Gefäß unter Siedekühlung und/oder Auskreisen des Reaktionswassers erfolgen.

In einer bevorzugten Ausführungsform wird das erfindungsgemäße Verfahren in einem diskontinuierlichen Mikrowellenreaktor durchgeführt. Dabei wird die Mikrowellenbestrahlung in einem gerührten Gefäß vorgenommen. Bevorzugt befinden sich zur Abführung überschüssiger Wärme im Reaktionsgefäß Kühlelemente wie beispielsweise Kühlfinger oder Kühlschlangen oder an das Reaktionsgefäß angeflanscht Rückflusskühler zur Siedekühlung des Reaktionsmediums. Für die Bestrahlung größerer Reaktionsvolumina wird die Mikrowelle hier bevorzugt im Multimode betrieben. Die diskontinuierliche Ausführungsform des erfindungsgemäßen Verfahrens erlaubt durch Variation der Mikrowellenleistung schnelle wie auch langsame Heizraten und insbesondere das Halten der Temperatur über längere Zeiträume wie beispielsweise mehrere Stunden. Die Reaktanden und gegebenenfalls Lösemittel und weitere Hilfsstoffe können vor Beginn der Mikrowellenbestrahlung im Reaktionsgefäß vorgelegt werden. Bevorzugt haben sie dabei Temperaturen unterhalb 100°C wie beispielsweise zwischen 10°C und 50°C. In einer bevorzugten Ausführungsform werden die Reaktanden oder Teile davon dem Reaktionsgefäß erst während der Bestrahlung mit Mikrowellen zugeführt. In einer weiteren bevorzugten Ausführungsform wird der diskontinuierliche Mikrowellenreaktor unter kontinuierlichem Zuführen von Edukten und gleichzeitigem Ausschleusen von Reaktionsgut in Form eines Semi-Batch- bzw. Kaskadenreaktors betrieben.

In einer besonders bevorzugten Ausführungsform wird das erfindungsgemäße Verfahren in einem kontinuierlichen Mikrowellenreaktor durchgeführt. Die Reaktionsmischung wird dazu durch ein druckfestes, gegenüber den Reaktanden inertes, für Mikrowellen weitestgehend transparentes und in einen Mikrowellenofen eingebautes Reaktionsrohr geführt. Dieses Reaktionsrohr hat bevorzugt einen Durchmesser von einem Millimeter bis ca. 50 cm, speziell zwischen 2 mm und 35 cm wie beispielsweise zwischen 5 mm und 15 cm. Unter Reaktionsrohren werden hier Gefäße verstanden, deren Verhältnis von Länge zu Durchmesser größer als 5, bevorzugt zwischen 10 und 100.000, besonders bevorzugt zwischen 20 und 10.000 wie beispielsweise zwischen 30 und 1.000 ist. In einer speziellen Ausführungsform ist das Reaktionsrohr in Form eines Doppelmantelrohres ausgestaltet, durch dessen Innen- und Außenraum die Reaktionsmischung nacheinander im Gegenstrom geführt werden kann, um beispielsweise die Temperaturführung und Energieeffizienz des Verfahrens zu erhöhen. Als Länge des Reaktionsrohres ist dabei die vom Reaktionsgemisch insgesamt durchströmte Strecke zu verstehen. Das Reaktionsrohr ist auf seiner Länge von mindestens einem, bevorzugt aber mehreren wie beispielsweise zwei, drei, vier, fünf, sechs, sieben acht oder mehr Mikrowellenstrahlem umgeben. Die Mikrowelleneinstrahlung erfolgt bevorzugt über den Rohrmantel. In einer weiteren bevorzugten Ausführungsform erfolgt die Mikrowelleneinstrahlung mittels mindestens einer Antenne über die Rohrenden. Das Reaktionsrohr ist üblicherweise am Einlass mit einer Dosierpumpe sowie einem Manometer und am Auslass mit einem Druckhalteventil und einem Wärmetauscher versehen. Bevorzugt werden die Edukte Amin und Fettsäure, beide unabhängig voneinander gegebenenfalls mit Lösemittel verdünnt, erst kurz vor dem Eintritt in das Reaktionsrohr vermischt. Weiterhin bevorzugt werden die Edukte dem erfindungsgemäßen Verfahren in flüssiger Form mit Temperaturen unterhalb 100°C und bevorzugt zwischen 10 und 95°C wie beispielsweise zwischen 20°C und 50°C zugeführt. Dazu können höher schmelzende Edukte beispielsweise in geschmolzenem Zustand oder mit Lösemittel versetzt eingesetzt werden. Ein Katalysator kann, sofern eingesetzt, einem der Edukte oder auch der Eduktmischung vor dem Eintritt in das Reaktionsrohr zugesetzt werden.

Durch Variation von Rohrquerschnitt, Länge der Bestrahlungszone (hierunter wird der Anteil des Reaktionsrohres verstanden, in dem das Reaktionsgut Mikrowellenstrahlung ausgesetzt ist), Fließgeschwindigkeit, Geometrie der Mikrowellenstrahler, der eingestrahlten Mikrowellenleistung sowie der dabei erreichten Temperatur werden die Reaktionsbedingungen so eingestellt, dass die maximale Reaktionstemperatur schnellstmöglich erreicht wird und die Verweilzeit bei Maximaltemperatur so kurz bleibt, dass so wenig Neben- oder Folgereaktionen wie möglich auftreten. Bevorzugt wird der kontinuierliche Mikrowellenreaktor im Monomode oder Quasi-Monomode betrieben. Die Verweilzeit im Reaktionsrohr liegt dabei im Allgemeinen unter 30 Minuten, bevorzugt zwischen 0,01 Sekunden und 15 Minuten und besonders bevorzugt zwischen 0,1 Sekunden und 5 Minuten wie beispielsweise zwischen einer Sekunde und 3 Minuten. Das Reaktionsgut kann zur Vervollständigung der Reaktion, gegebenenfalls nach Zwischenkühlung, mehrfach den Reaktor durchlaufen. Es hat sich besonders bewährt, wenn das Reaktionsprodukt unmittelbar nach Verlassen des Reaktionsrohres z. B. durch Mantelkühlung oder Entspannung abgekühlt wird.

Dabei war es besonders überraschend, dass trotz der im kontinuierlich durchflossenen Strömungsrohr nur sehr kurzen Verweildauer des Ammoniumsalzes im Mikrowellenfeld eine so weitgehende Amidierung ohne Bildung nennenswerter Mengen an Nebenprodukten stattfindet. Bei einer entsprechenden Umsetzung dieser Ammoniumsalze in einem Strömungsrohr unter thermischer Mantelheizung werden dagegen nur geringe Umsätze zum Amid erzielt. Gleichzeitig werden dabei jedoch durch die zur Erzielung geeigneter Reaktionstemperaturen benötigten extrem hohen Wandtemperaturen in erheblichem Ausmaß Zersetzungsreaktionen des Diamins und die Bildung gefärbter Spezies beobachtet.

Zur Vervollständigung der Umsetzung hat es sich in vielen Fällen bewährt, das erhaltene Rohprodukt nach Entfernen von Reaktionswasser sowie gegebenenfalls Austragen von Produkt und/oder Nebenprodukt erneut der Mikrowellenbestrahlung auszusetzen.

Üblicherweise fallen über den erfindungsgemäßen Weg hergestellte Amide in einer für die weitere Verwendung ausreichenden Reinheit an. Für spezielle Anforderungen können sie jedoch nach üblichen Reinigungsverfahren wie Destillation, Umkristallisation, Filtration bzw. chromatographische Verfahren weiter aufgereinigt werden.

Die erfindungsgemäß hergestellten basischen Amide eignen sich beispielsweise zur Herstellung von kationischen und insbesondere zur Herstellung von zwitterionischen Verbindungen. So führt die Quatemisierung mit Alkylhalogeniden wie beispielsweise Methyliodid, Methylbromid, Methylchlorid, Benzylbromid, Benzylchlorid oder Dimethylsulfat zu quartären, kationischen Strukturen. Auch durch oxalkylierende Quaternisierung können die erfindungsgemäß hergestellten basischen Amide in kationische Verbindungen überführt werden. Durch Umsetzung mit saure Gruppen tragenden Alkylierungsmitteln sind zwitterionische Strukturen (Betaine) zugänglich. Beispiele für geeignete saure Gruppen tragende Alkylierungsmittel sind Halogenessigsäuren bzw. deren Salze wie Chloressigsäure, Halogenalkansulfonsäuren bzw. deren Salze wie Bromethansulfonsäure sowie cyclische Sulfolane. Die Oxidation des tertiären Stickstoffs beispielsweise mit Peroxiden wie H₂O₂ führt zu N-Oxiden. All diese Strukturen sind oberflächenaktiv und finden vielfältige industrielle Verwendung zum Beispiel als Rohstoff für die Herstellung von Waschmitteln, Reinigungskonzentraten, Detergentien, Kosmetika und Pharmazeutika, als Emulgatoren sowie in der Erdölindustrie als Korrosions- oder Gashydratinhibitoren.

Das erfindungsgemäße Verfahren erlaubt eine sehr schnelle und kostengünstige Herstellung basischer Fettsäureamide in hohen Ausbeuten und mit hoher Reinheit. Die als Jodfarbzahl gemäß DIN 6162 gemessene Eigenfärbung der so hergestellten Amide (Konzentrate) liegt unter 5 und oftmals unter 4 wie beispielsweise unterhalb 3,5. Dabei fallen keine wesentlichen Mengen an Nebenprodukten an. Derartig schnelle und selektive Umsetzungen sind nach klassischen Methoden nicht zu erzielen und waren alleine durch Heizen auf hohe Temperaturen auch nicht zu erwarten. Da die nach dem erfindungsgemäßen Verfahren hergestellten basischen Amide sowie die aus ihnen abgeleiteten Verbindungen zudem verfahrensbedingt keine Reste von Kupplungsreagentien bzw. deren Folgeprodukten enthalten, können sie problemlos auch in toxikologisch sensiblen Bereichen wie beispielsweise kosmetischen und pharmazeutischen Zubereitungen eingesetzt werden. Bei der Wahl geeigneter Quaternisierungsmittel ist es ebenfalls möglich, von zu Korrosion führenden Halogenidionen freie Betaine bereitzustellen.

### Beispiele

Die Umsetzungen unter Mikrowellenbestrahlung erfolgten in einem Single-Mode Mikrowellenreaktor vom Typ "Discover" der Firma CEM bei einer Frequenz von 2,45 GHz. Die Kühlung der Reaktionsgefäße erfolgte mittels Druckluft. Die Temperaturmessung erfolgte über einen IR-Sensor am Küvettenboden. Die Temperaturmessungen mussten auf Grund der Druckbedingungen in den Reaktionsgefäßen über einen IR-Sensor am Küvettenboden erfolgen. Durch Vergleichsversuche mit einer in die Reaktionsmischung eintauchenden Glasfiberoptik wurde festgestellt, dass die Temperatur im Reaktionsmedium im hier relevanten Temperaturbereich etwa 50 bis 80°C über der mit dem IR-Sensor am Küvettenboden gemessenen Temperatur liegt.

Die diskontinuierlich durchgeführten Umsetzungen erfolgten in geschlossenen, druckfesten Glasküvetten mit einem Volumen von 8 ml unter Magnetrührung. Kontinuierlich durchgeführte Umsetzungen erfolgten in druckfesten, zylindrischen, als Doppelmantelrohr ausgestalteten Glasküvetten (ca. 10 x 1,5 cm; Reaktionsvolumen ca. 15 ml) mit innen liegendem, über dem Küvettenboden endendem Einleitungsrohr und Produktabnahme am oberen Ende der Küvette. Der sich während der Reaktion aufbauende Druck wurde über ein Druckhalteventil auf maximal 20 bar begrenzt und in eine Vorlage entspannt. Das Ammoniumsalz wurde durch das Einleitungsrohr in die Küvette gepumpt und die Verweilzeit in der Bestrahlungszone durch Modifizierung der Pumpenleistung eingestellt.

Die Analytik der Produkte erfolgte mittels ¹H-NMR-Spektroskopie bei 500 MHz in CDCl₃. Wasserbestimmungen erfolgten mittels Karl-Fischer-Titration. Die Jodfarbzahl wurde gemäß DIN 6162 bestimmt.

### Beispiel 1: Herstellung von N-(3-(N,N-Dimethylamino)propyl)capronamid

Unter Kühlen und Rühren wurde 1 g N,N-Dimethylaminopropylamin langsam mit einer äquimolaren Menge an Capronsäure versetzt und gemischt. Nach Abklingen der Wärmetönung wurde das so erhaltene Ammoniumsalz in einer geschlossenen Küvette für 1 Minute unter maximaler Kühlleistung einer Mikrowellenbestrahlung von 100 W ausgesetzt. Es wurde eine mittels IR-Sensor gemessene Temperatur von 195°C erreicht. Der sich während der Reaktion aufbauende Druck erreichte 15 bar.

Das erhaltene Rohprodukt enthielt als Hauptkomponenten 88 % N-(3-(N,N-Dimethylamino)propyl)capronamid, 7 % Wasser sowie unumgesetzte Edukte. Nach Trocknung des Reaktionsansatzes über Molekularsieb, erneute einminütige Bestrahlung mit Mikrowellen von 100 W und Trocknung über Molekularsieb wurde N-(3-(N,N-Dimethylamino)propyl)capronamid mit über 95 %iger Ausbeute erhalten. Die Jodfarbzahl betrug 3,0.

### Beispiel 2: Herstellung von N-(3-(N,N-Dimethylamino)propyl)laurylamid

Bei 50°C wurden 1,1 g N,N-Dimethylaminopropylamin unter Rühren langsam mit 2,0 g Laurinsäure versetzt und gemischt. Nach Abklingen der Wärmetönung wurde das so erhaltene Ammoniumsalz in einer geschlossenen Küvette für 1 Minute unter maximaler Kühlleistung einer Mikrowellenbestrahlung von 150 W ausgesetzt. Es wurde eine mittels IR-Sensor gemessene Temperatur von 150°C erreicht, wobei der Druck auf 3,5 bar stieg.

Das Rohprodukt enthielt als Hauptkomponente 90 % N-(3-(N,N-Dimethylamino)-propyl)laurylamid sowie 5 % Wasser und unumgesetzte Edukte. Nach Trocknung des Reaktionsansatzes über Molekularsieb, erneute einminütige Bestrahlung mit Mikrowellen von 100 W und Trocknung über Molekularsieb wurde N-(3-(N,N-Dimethylamino)propyl)laurylamid mit über 96 %iger Ausbeute erhalten. Die Jodfarbzahl betrug 1,8.

### Beispiel 3: Herstellung von N-(3-(N,N-Dimethylamino)propyl)caprylamid

Unter Kühlen und Rühren wurde 1 g N,N-Dimethylaminopropylamin langsam mit einer equimolaren Menge an Caprylsäure versetzt und gemischt. Nach Abklingen der Wärmetönung wurde das so erhaltene Ammoniumsalz in einer geschlossenen Küvette für 1 Minute unter maximaler Kühlleistung einer Mikrowellenbestrahlung von 100 W ausgesetzt. Es wurde eine mittels IR-Sensor gemessene Temperatur von 200°C erreicht wobei der Druck auf etwa 4 bar stieg.

Das Rohprodukt enthielt als Hauptkomponente 64 % N-(3-(N,N-Dimethylamino)-propyl)caprylamid, 7 % Wasser sowie unumgesetzte Edukte. Nach Trocknung des Reaktionsansatzes über Molekularsieb, erneute einminütige Bestrahlung mit Mikrowellen von 100 W und Trocknung über Molekularsieb wurde N-(3-(N,N-Dimethylamino)propyl)octylamid mit über 94 %iger Ausbeute erhalten. Die Jodfarbzahl betrug 2,2.

### Beispiel 4: Kontinuierliche Herstellung von N-(3-(N,N-Dimethylamino)propyl)caprylamid

Unter Kühlung und Rühren wurde eine Mischung aus 107 g (1,05 mol) N,N-Dimethylaminopropylamin und 100 g Xylol langsam mit 144 g (1 mol) Caprylsäure versetzt. Nach Abklingen der Wärmetönung wurde das so erhaltene Ammoniumsalz kontinuierlich über den Bodeneinlass durch die in der Mikrowellencavität montierte Glasküvette gepumpt. Die Förderleistung der Pumpe wurde dabei so eingestellt, dass die Verweilzeit in der Küvette und damit in der Bestrahlungszone etwa 50 Sekunden betrug. Es wurde unter maximaler Kühlleistung mit einer Mikrowellenleistung von 200 W gearbeitet, wobei eine mittels IR-Sensor gemessene Temperatur von 190°C erreicht wurde. Nach Verlassen der Glasküvette wurde die Reaktionsmischung über einen kurzen Liebig-Kühler auf 35°C abgekühlt..

Nach Abtrennen des Reaktionswassers wurde das Rohprodukt nochmals wie oben durch die Glasküvette gepumpt und dabei erneut Mikrowellenstrahlung ausgesetzt. Nach Abdestillieren von Xylol, überschüssigem Dimethylaminoproplymamin und Reaktionswasser wurde N-(3-(N,N-Dimethylamino)propyl)caprylamid mit 92 %iger Ausbeute erhalten. Die Jodfarbzahl betrug 1,2.

### Beispiel 5: Kontinuierliche Herstellung von N-(3-(N,N-Dimethylamino)-propyl)-cocosfettsäureamid

Unter Kühlung und Rühren wurden 122 g (1,2 mol) N,N-Dimethylaminopropylamin langsam mit 214 g (1 mol) geschmolzener Cocosfettsäure (Gemisch aus hauptsächlich C₁₂- und C₁₄-Fettsäure) versetzt. Nach Abklingen der Wärmetönung wurde das so erhaltene Ammoniumsalz kontinuierlich durch die in der Mikrowellencavität montierte Glasküvette gepumpt. Die Förderleistung der Pumpe wurde dabei so eingestellt, dass die Verweilzeit in der Küvette und damit in der Bestrahlungszone etwa 60 Sekunden betrug. Es wurde unter maximaler Kühlleistung mit einer Mikrowellenleistung von 200 W gearbeitet, wobei eine mittels IR-Sensor gemessene Temperatur von 190 °C erreicht wurde. Nach Verlassen der Glasküvette wurde die Reaktionsmischung über einen kurzen Liebig-Kühler auf 35°C abgekühlt.

Nach Abtrennen des Reaktionswassers wurde das Rohprodukt nochmals wie oben durch die Glasküvette gepumpt und dabei erneut Mikrowellenstrahlung ausgesetzt. Nach Abdestillieren von überschüssigem N,N-Dimethylaminopropylamin und Reaktionswasser wurde N-(3-(N,N-Dimethylamino)propyl)-cocosfettsäureamid mit 96 %iger Ausbeute erhalten. Die Jodfarbzahl betrug 0,9.

### Beispiel 6: Kontinuierliche Herstellung von N-(3-(N,N-Dimethylamino)propyl)laurinsäureamid

Unter Kühlung und Rühren wurden 102 g (1 mol) N,N-Dimethylaminopropylamin langsam mit 214 g (1 mol) geschmolzener Laurinsäure versetzt. Nach Abklingen der Wärmetönung wurde das so erhaltene Ammoniumsalz kontinuierlich durch die in der Mikrowellencavität montierte Glasküvette gepumpt. Die Förderleistung der Pumpe wurde dabei so eingestellt, dass die Verweilzeit in der Küvette und damit in der Bestrahlungszone etwa 70 Sekunden betrug. Es wurde unter maximaler Kühlleistung mit einer Mikrowellenleistung von etwa 200 W gearbeitet, wobei eine mittels IR-Sensor gemessene Temperatur von 195°C erreicht wurde. Nach Verlassen der Glasküvette wurde die Reaktionsmischung über einen kurzen Liebig-Kühler auf ungefähr 70°C (> Schmelzpunkt des Produktes) abgekühlt.

Nach Abtrennen des Reaktionswassers im Vakuum wurde das Rohprodukt nochmals wie oben durch die Glasküvette gepumpt und dabei erneut Mikrowellenstrahlung ausgesetzt. Nach Abdestillieren von nicht umgesetztem N,N-Dimethylaminopropylamin und Reaktionswasser wurde N-(3-(N,N-Dimethylamino)propyl)laurinsäureamid mit 94 %iger Ausbeute erhalten. Die Jodfarbzahl betrug 1,0.

### Beispiel 7: Kontinuierliche Herstellung von N-(3-(N,N-Diethylamino)propyl)laurinsäureamid

Unter Kühlung und Rühren wurden 133 g (1,02 mol) N,N-Diethylaminopropylamin langsam mit 214 g (1 mol) geschmolzener Laurinsäure versetzt. Nach Abklingen der Wärmetönung wurde das so erhaltene Ammoniumsalz kontinuierlich durch die in der Mikrowellencavität montierte Glasküvette gepumpt. Die Förderleistung der Pumpe wurde dabei so eingestellt, dass die Verweilzeit in der Küvette und damit in der Bestrahlungszone etwa 75 Sekunden betrug. Es wurde unter maximaler Kühlleistung mit einer Mikrowellenleistung von etwa 300 W gearbeitet, wobei eine mittels IR-Sensor gemessene Temperatur von 200°C erreicht wurde. Nach Verlassen der Glasküvette wurde die Reaktionsmischung über einen kurzen Liebig-Kühler auf ungefähr 70°C (> Schmelzpunkt des Produktes) abgekühlt. Anschließend wurde im Vakuum das Reaktionswassers entfernt und das Rohprodukt nochmals wie oben beschrieben durch die Glasküvette gepumpt und dabei erneut Mikrowellenstrahlung mit einer Leistung von 300 Watt ausgesetzt. Insgesamt wird der Vorgang dreimal wiederholt und zum Abschluss alle niedrigsiedende Anteile (vornehmlich Reaktionswasser und Spuren N,N-Diethylaminopropylamin) per Vakuumdestillation entfernt. Es wurde N-(3-(N,N-Diethylamino)propyl)laurinsäureamid mit 98,5 %iger Ausbeute erhalten. Die Jodfarbzahl betrug 1,0.

### Beispiel 8: Herstellung von N-(3-(N,N-Dimethylamino)propyl)cocosfettsäureamid (Vergleichsbeispiel)

In einem 1 Liter Dreihalskolben mit Wasserabscheider, KPG Rührer und Tropftrichter wurden 214 g (1 mol Cocosfettsäure, Gemisch aus hauptsächlich C₁₂- und C₁₄-Fettsäure) und 2g Methansulfonsäure als Katalysator vorgelegt, mit Stickstoff überlagert und bis zum Schmelzen erhitzt. Sobald die Schmelze homogen war, wurden langsam 113 g (1,1 mol) Dimethylaminopropylamin zugegeben. Dabei kam es auf Grund der Neutralisationsreaktion zu einem sehr deutlichen Temperaturanstieg. Sobald die exotherme Reaktion abgeklungen war, wurde die Reaktionsmischung zum Rückfluss erhitzt und Reaktionswasser ausgekreist. Die Innentemperatur des Reaktionsgemisches stieg mit fortschreitender Reaktion auf 180 -185°C an. Nach 15 Stunden wurde kein weiteres Reaktionswasser mehr abgeschieden und das Reaktionsgemisch wurde destillativ von überschüssigem N,N-Dimethylaminopropylamin und restlichen Reaktionswasser befreit.

Nach Abkühlen wurden 329 g N-(3-(N,N-Dimethylamino)propyl)cocosfettsäure-amid (89 % d. Th.) mit einer Jodfarbzahl von 7,5 erhalten

### Beispiel 9: Kontinuierliche Herstellung von N-(3-(N,N-Dimethylamino)propyl)-laurinsäureamid (Vergleichsbeispiel)

Unter Kühlung und Rühren wurden 75 g Propanolamin (1 mol) bei 40°C langsam mit 214 g (1 mol) Laurinsäure versetzt. Nach Abklingen der Wärmetönung wurde das so erhaltene Ammoniumsalz kontinuierlich über den Bodeneinlass durch die in einem 250°C heißen Ölbad platzierte, druckfeste Glasküvette gepumpt. Die Förderleistung der Pumpe wurde dabei so eingestellt, dass die Verweilzeit in der Küvette ungefähr 2 Minuten betrug. Die am Überlauf gemessene Temperatur erreichte dabei maximal 175°C. Nach Verlassen der Glasküvette wurde die Reaktionsmischung über einen kurzen Liebig-Kühler auf etwa 70°C abgekühlt und danach das Reaktionswasser im Vakuum abgetrennt. Mittels Karl Fischer Titration wurden danach lediglich Spuren von Wasser nachgewiesen (< 0,2 %), woraufhin das Rohprodukt nochmals wie oben beschrieben durch die Glasküvette gepumpt und dabei erneut innerhalb des Intervalls von 2 Minuten thermischer Energie ausgesetzt wurde. Im Umsetzungsprodukt konnte nur eine geringe Menge an Laurinsäurepropanolamid von etwa 4 % nachgewiesen werden. Nebenprodukte konnten aufgrund der sehr geringen Umsetzungsrate im ¹H-NMR-Spektrum nicht identifiziert werden.
Das Reaktionsgemisch besaß nach der angewandten Reaktionsfolge eine Jodfarbzahl von 5,9.

## Patentansprüche

1. Verfahren zur Herstellung von basischen Fettsäureamiden, indem mindestens ein Amin, das mindestens eine primäre oder sekundäre Aminogruppe und mindestens eine tertiäre Aminogruppe enthält, mit mindestens einer Fettsäure zu einem Ammoniumsalz umgesetzt wird, und dieses Ammoniumsalz nachfolgend unter Mikrowellenbestrahlung weiter zum basischen Amid umgesetzt wird.

2. Verfahren nach Anspruch 1, worin die Fettsäure einen Kohlenwasserstoffrest mit 1 bis 50 Kohlenstoffatomen umfasst.

3. Verfahren nach Anspruch 2, worin die Fettsäure einen aliphatischen Kohlenwasserstoffrest mit 6 bis 30 Kohlenstoffatomen umfasst.

4. Verfahren nach einem oder mehreren der Ansprüche 1 bis 3, worin der Kohlenwasserstoffrest der Fettsäure mindestens einen Substituenten ausgewählt aus Halogenatomen, halogenierten Alkylresten, Cyano-, Hydroxyalkyl-, Hydroxyl-, Methoxy-, Nitril-, Nitro-, und Sulfonsäuregruppen umfasst.

5. Verfahren nach einem oder mehreren der Ansprüche 1 bis 4, worin der Kohlenwasserstoffrest der Fettsäure gesättigt ist.

6. Verfahren nach einem oder mehreren der Ansprüche 1 bis 4, worin der Kohlenwasserstoffrest der Fettsäure mindestens eine Doppelbindung umfasst.

7. Verfahren nach einem oder mehreren der Ansprüche 1 bis 6, worin die Fettsäure aus der Gruppe bestehend aus Octan-, Decan-, Dodecan-, Tridecan-, Tetradecan-, 12-Methyltridecan-, Pentadecan-, 13-Methyltetradecan-, 12-Methyltetradecan-, Hexadecan-, 14-Methylpentadecan-, Heptadecan-, 15-Methylhexadecan-, 14-Methylhexadecan-, Octadecan-, Iso-Octadecan-, Icosan-, Docosan- und Tetracosan-, Myristolein-, Palmitolein-, Hexadecadien-, Delta-9-cis-Heptadecen-, Öl-, Petroselin-, Vaccen-, Linol-, Linolen-, Gadolein-, Gondo-, Icosadien-, Arachidon-, Cetolein-, Eruca-, Docosadien-, Tetracosen-, Ricinol-, Tallölfett-, Harz- und Naphthensäuren ausgewählt ist.

8. Verfahren nach einem oder mehreren der Ansprüche 1 bis 7, worin das Amin eine primäre Aminogruppe enthält.

9. Verfahren nach einem oder mehreren der Ansprüche 1 bis 8, worin das Amin drei oder mehr Aminogruppen enthält, von denen mindestens eine primär, mindestens eine sekundär und mindestens eine tertiär ist.

10. Verfahren nach einem oder mehreren der Ansprüche 1 bis 9, worin das Amin der Formen
HNR¹-(A)ₙ-Z
entspricht, worin
R¹ für Wasserstoff, C₁-C₁₂-Alkyl, C₅-C₁₂-Cycloalkyl, C₆-C₁₂-Aryl, C₇-C₁₂-Aralkyl oder eine heteroaromatische Gruppe mit 5 bis 12 Ringgliedern,
A für einen Alkylenrest mit 1 bis 12 C-Atomen, einen Cycloalkylenrest mit 5 bis 12 Ringgliedern, einen Arylenrest mit 6 bis 12 Ringgliedern oder einen Heteroarylenrest mit 5 bis 12 Ringgliedern
n für 0 oder 1,
Z für eine Gruppe der Formel -NR²R³ oder für einen Stickstoff enthaltenden zyklischen Kohlenwasserstoffrest mit mindestens 5 Ringgliedern und
R², R³ unabhängig voneinander für C₁- bis C₂₀-Kohlenwasserstoffreste stehen.

11. Verfahren nach einem oder mehreren der Ansprüche 1 bis 10, wobei die Mikrowellenbestrahlung in Gegenwart eines dehydratisierenden Katalysators durchgeführt wird.

12. Verfahren nach einem oder mehreren der Ansprüche 1 bis 11, wobei die Mikrowellenbestrahlung in Gegenwart eines Lösemittels durchgeführt wird.

13. Verfahren nach Anspruch 12, wobei das Lösemittel einen ε"-Wert von unter 10 aufweist.

14. Verfahren nach einem oder mehreren der Ansprüche 1 bis 13, wobei die Mikrowellenbestrahlung bei Temperaturen unterhalb 300°C durchgeführt wird.

15. Verfahren nach einem oder mehreren der Ansprüche 1 bis 14, wobei die Reaktion bei Drücken zwischen 0,1 und 200 bar durchgeführt wird.

16. Verfahren nach einem oder mehreren der Ansprüche 1 bis 15, wobei die Umsetzung kontinuierlich durch Bestrahlung mit Mikrowellen in einem vom Ammoniumsalz durchströmten Reaktionsrohr erfolgt.

17. Verfahren nach Anspruch 16, wobei das Reaktionsrohr aus einem nicht metallischen, mikrowellentransparenten Werkstoff besteht.

18. Verfahren nach Anspruch 16 und/oder 17, worin die Verweilzeit des Reaktionsgutes im Reaktionsrohr unter 30 Minuten beträgt.

19. Verfahren nach einem oder mehreren der Ansprüche 16 bis 18, wobei das Reaktionsrohr ein Verhältnis von Länge zu Durchmesser von mindestens 5 besitzt.

## Claims

1. A process for preparing basic fatty acid amides by reacting at least one amine which contains at least one primary or secondary amino group and at least one tertiary amino group with at least one fatty acid to give an ammonium salt, and then converting this ammonium salt further under microwave irradiation to the basic amide.

2. The process as claimed in claim 1, in which the fatty acid comprises a hydrocarbon radical having 1 to 50 carbon atoms.

3. The process as claimed in claim 2, in which the fatty acid comprises an aliphatic hydrocarbon radical having 6 to 30 carbon atoms.

4. The process as claimed in one or more of claims 1 to 3, in which the hydrocarbon radical of the fatty acid comprises at least one substituent selected from halogen atoms, halogenated alkyl radicals, or cyano, hydroxyalkyl, hydroxyl, methoxy, nitrile, nitro and sulfonic acid groups.

5. The process as claimed in one or more of claims 1 to 4, in which the hydrocarbon radical of the fatty acid is saturated.

6. The process as claimed in one or more of claims 1 to 4, in which the hydrocarbon radical of the fatty acid comprises at least one double bond.

7. The process as claimed in one or more of claims 1 to 6, in which the fatty acid is selected from the group consisting of octanoic acid, decanoic acid, dodecanoic acid, tridecanoic acid, tetradecanoic acid, 12-methyltridecanoic acid, pentadecanoic acid, 13-methyltetradecanoic acid, 12-methyltetradecanoic acid, hexadecanoic acid, 14-methylpentadecanoic acid, heptadecanoic acid, 15-methylhexadecanoic acid, 14-methylhexadecanoic acid, octadecanoic acid, isooctadecanoic acid, eicosanoic acid, docosanoic acid and tetracosanoic acid, myristoleic acid, palmitoleic acid, hexadecadienoic acid, delta-9-cis-heptadecenoic acid, oleic acid, petroselic acid, vaccenic acid, linoleic acid, linolenic acid, gadoleic acid, gondoleic acid, eicosadienoic acid, arachidonic acid, cetoleic acid, erucic acid, docosadienoic acid, tetracosenoic acid, ricinoleic acid, tall oil fatty acid, resin acids and naphthenic acids.

8. The process as claimed in one or more of claims 1 to 7, in which the amine contains a primary amino group.

9. The process as claimed in one or more of claims 1 to 8, in which the amine contains three or more amino groups, of which at least one is primary, at least one is secondary and at least one is tertiary.

10. The process as claimed in one or more of claims 1 to 9, in which the amine corresponds to the formula
HNR¹-(A)ₙ-Z
in which
R¹ is hydrogen, C₁-C₁₂-alkyl, C₅-C₁₂-cycloalkyl, C₆-C₁₂-aryl, C₇-C₁₂-aralkyl or a heteroaromatic group having 5 to 12 ring members,
A is an alkylene radical having 1 to 12 carbon atoms, a cycloalkylene radical having 5 to 12 ring members, an arylene radical having 6 to 12 ring members or a heteroarylene radical having 5 to 12 ring members,
n is 0 or 1,
Z is a group of the formula -NR²R³ or a nitrogen-containing cyclic hydrocarbon radical having at least 5 ring members and
R², R³ are each independently C₁- to C₂₀-hydrocarbon radicals.

11. The process as claimed in one or more of claims 1 to 10, wherein the microwave irradiation is performed in the presence of a dehydrating catalyst.

12. The process as claimed in one or more of claims 1 to 11, wherein the microwave irradiation is performed in the presence of a solvent.

13. The process as claimed in claim 12, wherein the solvent has an ε" value of less than 10.

14. The process as claimed in one or more of claims 1 to 13, wherein the microwave irradiation is performed at temperatures below 300°C.

15. The process as claimed in one or more of claims 1 to 14, wherein the reaction is performed at pressures between 0.1 and 200 bar.

16. The process as claimed in one or more of claims 1 to 15, wherein the reaction is effected continuously by irradiating with microwaves in a reaction tube through which the ammonium salt flows.

17. The process as claimed in claim 16, wherein the reaction tube consists of a nonmetallic microwave-transparent material.

18. The process as claimed in claim 16 and/or 17, in which the residence time of the reaction mixture in the reaction tube is less than 30 minutes.

19. The process as claimed in one or more of claims 16 to 18, wherein the reaction tube has a ratio of length to diameter of at least 5.

## Revendications

1. Procédé de préparation d'amides d'acides gras basiques, dans lequel transforme une amine, qui contient au moins un groupe amino primaire ou secondaire et au moins un groupe amino tertiaire, avec au moins un acide gras en un sel d'ammonium et on transforme ce sel d'ammonium ensuite davantage sous une irradiation par des micro-ondes en amide basique.

2. Procédé selon la revendication 1, l'acide gras comprenant un radical hydrocarboné comprenant 1 à 50 atomes de carbone.

3. Procédé selon la revendication 2, l'acide gras comprenant un radical hydrocarboné aliphatique comprenant 6 à 30 atomes de carbone.

4. Procédé selon l'une ou plusieurs des revendications 1 à 3, le radical hydrocarboné de l'acide gras comprenant au moins un substituant choisi parmi les atomes d'halogène, les radicaux alkyle halogénés, les groupes cyano, hydroxyalkyle, hydroxyle, méthoxy, nitrile, nitro et acide sulfonique.

5. Procédé selon l'une ou plusieurs des revendications 1 à 4, le radical hydrocarboné de l'acide gras étant saturé.

6. Procédé selon l'une ou plusieurs des revendications 1 à 4, le radical hydrocarboné de l'acide gras comprenant au moins une double liaison.

7. Procédé selon l'une ou plusieurs des revendications 1 à 6, l'acide gras étant choisi dans le groupe constitué par l'acide octanoïque, décanoïque, dodécanoïque, tridécanoïque, tétradécanoïque, 12-méthyltridécanoïque, pentadécanoïque, 13-méthyltétradécanoïque, 12-méthyltétradécanoïque, hexadécanoïque, 14-méthylpentadécanoïque, heptadécanoïque, 15-méthylhexadécanoïque, 14-méthylhexadécanoïque, octadécanoïque, iso-octadécanoïque, icosanoïque, docosanoïque et tétracosanoïque, myristoléique, palmitoléique, hexadécadiénoïque, delta-9-cis-heptadécénoïque, oléique, pétrosélique, vaccénique, linoléique, linolénique, gadoléique, gondoïque, icosadiénoïque, arachidonique, cétoléïque, érucasique, docosadiénoïque, tétracosénoïque, ricinoléique, l'acide gras de tall oil, l'acide résinique et l'acide naphténique.

8. Procédé selon l'une ou plusieurs des revendications 1 à 7, l'amine contenant un groupe amino primaire.

9. Procédé selon l'une ou plusieurs des revendications 1 à 8, l'amine contenant trois groupes amino ou plus, dont au moins un est primaire, au moins un est secondaire et au moins un est tertiaire.

10. Procédé selon l'une ou plusieurs des revendications 1 à 9, l'amine présentant la formule
HNR¹-(A)ₙ-Z
où
R¹ représente hydrogène, C₁-C₁₂-alkyle, C₅-C₁₂-cycloalkyle, C₆-C₁₂-aryle, C₇-C₁₂-aralkyle ou un groupe hétéroaromatique comprenant 5 à 12 chaînons de cycle,
A représente un radical alkylène comprenant 1 à 12 atomes de carbone, un radical cycloalkylène comprenant 5 à 12 chaînons de cycle, un radical arylène comprenant 6 à 12 chaînons de cycle ou un radical hétéroarylène comprenant 5 à 12 chaînons de cycle
n vaut 0 ou 1,
Z représente un groupe de formule -NR²R³ ou un radical hydrocarboné cyclique contenant de l'azote comprenant au moins 5 chaînons de cycle et
R², R³ représentent, indépendamment l'un de l'autre, des radicaux hydrocarbonés en C₁-C₂₀.

11. Procédé selon l'une ou plusieurs des revendications 1 à 10, l'irradiation par des micro-ondes étant réalisée en présence d'un catalyseur de déshydratation.

12. Procédé selon l'une ou plusieurs des revendications 1 à 11, l'irradiation par des micro-ondes étant réalisée en présence d'un solvant.

13. Procédé selon la revendication 12, le solvant présentant une valeur ε" inférieure à 10.

14. Procédé selon l'une ou plusieurs des revendications 1 à 13, l'irradiation par des micro-ondes étant réalisée à des températures inférieures à 300°C.

15. Procédé selon l'une ou plusieurs des revendications 1 à 14, la réaction étant réalisée à des pressions entre 0,1 et 200 bars.

16. Procédé selon l'une ou plusieurs des revendications 1 à 15, la transformation ayant lieu en continu par irradiation par des micro-ondes dans un tube de réaction traversé par un sel d'ammonium.

17. Procédé selon la revendication 16, le tube de réaction étant constitué d'un matériau non métallique, transparent pour les micro-ondes.

18. Procédé selon la revendication 16 et/ou 17, le temps de séjour du produit de réaction dans le tube de réaction étant inférieur à 30 minutes.

19. Procédé selon l'une ou plusieurs des revendications 16 à 18, le tube de réaction présentant un rapport longueur à diamètre d'au moins 5.
